# EUROPÄISCHE PATENTSCHRIFT

(11) **EP 0 622 062 B1**
(45) Veröffentlichungstag und Bekanntmachung des Hinweises auf die Patenterteilung: **04.06.1997**
(21) Anmeldenummer: 94106165.7
(22) Anmeldetag: 21.04.1994
(51) Int. Cl.: A61F 2/40

(54) **Schultergelenk-Endoprothese**
Shoulder prothesis
Prothèse de l'épaule

(30) Priorität: 30.04.1993 DE 4314200
(43) Veröffentlichungstag der Anmeldung: 02.11.1994
(73) Patentinhaber: ESKA Implants GmbH & Co., 23556 Lübeck (DE)
(72) Erfinder: Grundei, Hans, D-23558 Lübeck (DE)
(74) Vertreter: Fuchs, Luderschmidt & Partner Patentanwälte

(56) Entgegenhaltungen:
- FR-A- 2 430 221
- GB-A- 1 302 834
- GB-A- 2 001 247
- US-A- 4 030 143

## Beschreibung

Die Erfindung betrifft eine Schultergelenk-Endoprothese, die aus einem im Oberarmknochen (Humerus) zu verankernden Stielteil, aus einem mit dem proximalen Ende des Stielteils verbindbaren Kugelgelenkkopf und aus einem in der natürlichen Gelenkpfanne (Cavitas glenoidalis) am Schulterblatt fixierbaren Lager.

Bei einem natürlichen Schultergelenk ist der natürliche Gelenkkopf in der Cavitas glenoidalis gelagert und wird durch eine Rotatorenmanschette, die die Sehne des Musculus Supraspinatus bildet, vor einer Luxation aus der Cavitas glenoidalis bewahrt. Bei einer Zerstörung dieser natürlichen Manschette kommt es zur Luxation des Gelenkkopfes aus der Cavitas glenoidalis. Die gleichen Symptome sind zu verzeichnen bei Rupturen von Muskelfasern in diesem Bereich. Kommt es beispielsweise nach einem Tumorbefall zur Resektion des natürlichen Gelenks stellt sich die Frage, wie denn der Gelenkkopf des künstlichen Gelenks in dem künstlichen Lager gehalten werden kann, wenn nicht etwas zur Rotatorenmanschette Vergleichbares vorgesehen sein kann, da diese ebenfalls resiziert werden muß.

Bekannte Endoprothesen für das Schultergelenk sind ersichtlich beispielsweise aus der DE-91 15 283 Ul, der US-A-4,045,825, der US-A-4,045,826 und der US-A-4,030,143. Aus der letztgenannten ist ein zweischaliger Aufbau des Gelenklagers ersichtlich. Dieses Gelenklager umhüllt die Gelenkkugel mit Ausnahme einer Aussparung, durch welche der Hals der Gelenkkugel greift, vollständig. Aus den beiden vorstehend erwähnten anderen US-Druckschriften ist eine Schultergelenk-Endoprothese bekannt, bei der das Pfannenteil halbkalottenförmig mit einigen im Schulterblatt einzusetzenden, von der Oberfläche der Halbkalotte wegragenden Zapfen versehen sind. Schließlich ist aus der erwähnten DE-U1 ebenfalls eine Schultergelenk-Endoprothese bekannt, bei der der Gelenkkopf distal unterhalb seines größten Durchmessers eine zumindest teilweise umlaufende Nut aufweist zur Aufnahme eines den Kopf umschlingenden Bandes aus körperverträglichem Material.

Fraglich bei allen diesen Endoprothesen ist die exakte Positionierung des künstlichen Kugelgelenkkopfes in der Gelenkpfanne. So ist einer Luxation im Falle der US-A-4,005,825 bzw. '826 durch keinerlei konstruktive Merkmale vorgebeugt. Im Falle der DE-U1 versucht man dies zu verhindern durch das den Kopf umschlingende Band. Im Falle der US-A-4,030,143 kann zwar der Kugelgelenkkopf aus dem zweischaligen Gelenklager nicht ohne weiteres luxieren. Fraglich allerdings erscheint die Stabilität der Lage des Gelenklagers selbst in der Cavitas glenoidalis.

Eine gattungsgemäße Endoprothese, die auch als Ersatz eines Schultergelenkes dienen kann, ist bekannt geworden aus der FR-A-2430221. Neben den eingangs erwähnten Merkmalen ist darüber hinaus gemäß diesem Vorschlag vorgesehen, das Lager als eine Art Lagerkäfig für den Kugelgelenkkopf auszubilden. Konkrete Vorschläge aus dieser Druckschrift sehen vor, im Inneren besonders gestaltete kalottenförmige Gelenkpfannen als Lagerkäfig auszubilden, in welche der Kugelgelenkkopf in einer Auslenkstellung setzbar ist, um hiernach nach Verschwenkung in eine Art Verriegelungsstellung zu gelangen. Dabei ist vorgesehen, daß durch Verschwenkung des Kugelgelenkkopfes bei den üblichen physiologischen Bewegungen dieser nicht mehr in die Entriegelungsstellung gelangen kann, womit eine Luxation des Gelenkkopfes verhindert werden soll.

Die Aufgabe der vorliegenden Erfindung ist vor dem aufgezeigten Hintergrund darin zu sehen, eine gattungsgemäße Schultergelenk-Endoprothese so weiterzubilden, bei der eine absolut sichere Positionierung des künstlichen Kugelgelenkkopfes gewährleistet ist. Die Endoprothese soll darüber hinaus selbstverständlich die physiologischen Armbewegungen zulassen.

Gelöst wird diese Aufgabe durch eine Schultergelenk-Endoprothese mit dem Merkmalen, wie sie Kennzeichnenden Teil des Anspruchs 1 angegeben sind. Vorteilhafte Ausgestaltungen und Weiterbildungen ergeben sich aus den Ansprüchen 2 bis 6.

Es handelt sich bei der erfindungsgemäßen Endoprothese um eine sogenannte Vollendoprothese, bei der also alle natürlichen Gelenkteile resiziert und durch ein Implantat ersetzt wird.

Die Schultergelenk-Endoprothese besteht aus einem Stielteil, dessen Stiel im Humerus verankerbar ist. Am proximalen Ende des Stielteils ist mit diesem ein Kugelgelenkkopf verbindbar, beispielsweise über eine konische Verklemmung. Das Gegenlager für den Kugelgelenkkopf ist gebildet aus einem käfigartigen Lager, das in der natürlichen Gelenkpfanne (Cavitas glenoidalis) verankerbar ist. Hierzu wird die Cavitas glenoidalis üblicherweise angefrischt durch ein Anfräsen zur Herstellung definierter Anlageflächen etc..

Erfindungsgemäß übergreift das käfigartige Lager den Kugelgelenkkopf über dessen Äquatorlinie und sichert so die Position der Kugel im Lager. Hierzu ist vorgesehen, daß der Lagerkäfig aus im wesentlichen drei Ringsegmenten besteht. Das erste Ringsegment weist eine solche Bogenlänge auf, die größer ist als der halbe Kreisumfang, dessen Teil das Segment selbst ist. Die beiden anderen Ringsegmente sind an den Enden des ersten Ringsegmentes so angebracht, daß sie die Äquatorlinie des Kugelgelenkkopfes zumindest teilweise übergreifen. Hierbei sind die Radien der Ringsegmente und des Kugelgelenkkopfes im wesentlichen gleich. Wenn die beiden anderen Ringsegmente senkrecht an den Enden des ersten Ringsegmentes angebracht sind, so übergreifen die beiden anderen Ringsegmente die Äquatorlinie des Kugelgelenkkopfes vollständig. Das erste Ringsegment bietet gewissermaßen die Basis des Lagerkäfigs und liegt mit seiner Außenseite nach der Implantation an der angefrischten Cavitas glenoidalis an.

Die erfindunsgemäße Konstruktion des Lagerkäfigs gewährleistet eine dauerhafte und sichere Lagerung des Kugelgelenkkopfes im Lager, ohne daß die natürliche Rotatorenmanschette beispielsweise durch ein elastisches Band zwischen Lagerkäfig und Stielteil der Endoprothese nachempfunden würde.

Vorzugsweise sind die dem Kugelgelenkkopf zugewandten Flächen des Lagerkäfigs mit jeweils einem Inlay aus reibungsarmem Material belegt. Dies muß so elastisch sein, daß der Kugelgelenkkopf unter Verformung des Inlays in den Kugelkäfig eingedrückt werden kann. Beispielweise kommt hier als Material hochverdichtetes Polyethylen in Betracht. Gemäß einer bevorzugten Ausführungsform sind die Ringsegmente des Lagerkäfigs aus körperverträglichem Metall gegossen, die dann mit einer Polyethylenschicht belegt werden. Um die Rückhaltekraft noch zu erhöhen, ist vorteilhaft vorgesehen, daß die Inlays der beiden anderen Ringsegmente auf ihrer Außenfläche eine Nut aufweisen, in welche ein offener Federring eingelegt ist. Dieser wirkt nach Einführung des Kugelgelenkkopfes in den Lagerkäfig quasi wie eine Sprengring und erhöht die Sicherheit gegen ein Luxieren des Kopfes aus dem Lager.

Diese Ausführungsform wird noch dadurch verbessert, wenn die beiden anderen Ringsegmente auf Ihrer Innenseite eine Nut aufweisen, in welche der besagte Federring zu liegen kommt. Der Feder- oder Sprengring kommt also zwischer den Außenflächen der Inlays und den Innenseiten der metallischen beiden anderen Ringsegmente zu liegen.

Die vorstehend erläuterten konstruktiven Maßnahmen dienen ausschließlich zur dauerhaften Positionierung des Kugelgelenkkopfes in dem Lagerkäfig. Die beiden nachfolgend aufgeführten Weiterbildungen dienen zur dauerhaften Fixierung des Lagerkäfigs selbst am Schlüsselbein.

Gemäß einer vorteilhaften Weiterbildung ist vorgesehen, daß am ersten Ringsegment, also dem Basissegment, außen im Bereich des einen anderen Ringsegmentes ein in etwa tangential angesetzter Flansch als Arretierungsmittel des Lagerkäfigs angeformt ist. Dieser angesetzte Flansch kann während der Operation über den vorstehenden Teil des Schulterplatte, dem Acromion, gedreht werden mit einer Eindrehbewegung des Lagerkäfigs und so zu einer Art Klemmsitz des Lagerkäfigs in der angefrischten Cavitas glenoidalis führen.

Als weiteres vorteilhaftes Dauerarretierungsmittel ist beim ersten Ringsegment ein radial nach außen ragender Verankerungszapfen vorgesehen.

Die Erfindung wird nachstehend anhand eines Ausführungsbeispiels der Schultergelenk-Endoprothese gemäß der anliegenden Zeichnungen beispielhaft näher erläutert. Hierbei zeigt:
- Fig. 1: eine schematische Ansicht der kompletten Schultergelenk-Endoprothese nach der Implantation,
- Fig. 2: eine Schnittansicht des Lagerkäfigs mit darin gelagertem Kugelgelenkkopf, geschnitten in der Ebene des ersten Ringsegments und
- Fig. 3: eine Ansicht des Lagerkäfigs mit darin gelagerter Kugelgelenkpfanne von hinten auf das erste Ringsegment.

In den Zeichnungen sind gleiche Teile mit denselben Bezugszeichen versehen.

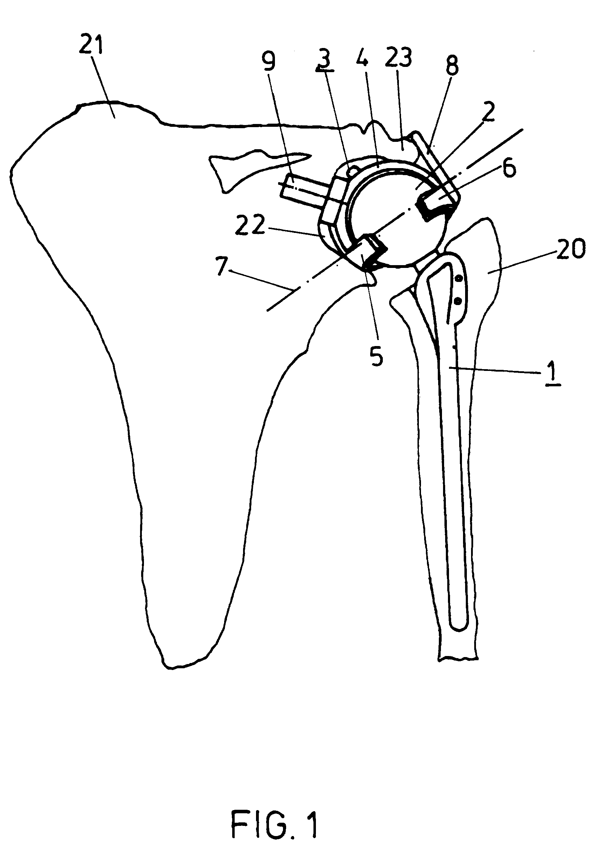

Fig. 1 zeigt schematisch den Sitz der implantierten Schultergelenk-Endoprothese am Schulterblatt 21 bzw. am Oberarmknochen (Humerus) 20. Das Stielteil 1 ist in geeigneter Weise im Humerus 20 verankert. An seinem proximalen Ende ist ein Kugelgelenkkopf 2 aufgesetzt und wird dort beispielsweise durch eine konische Verklemmung gehalten.

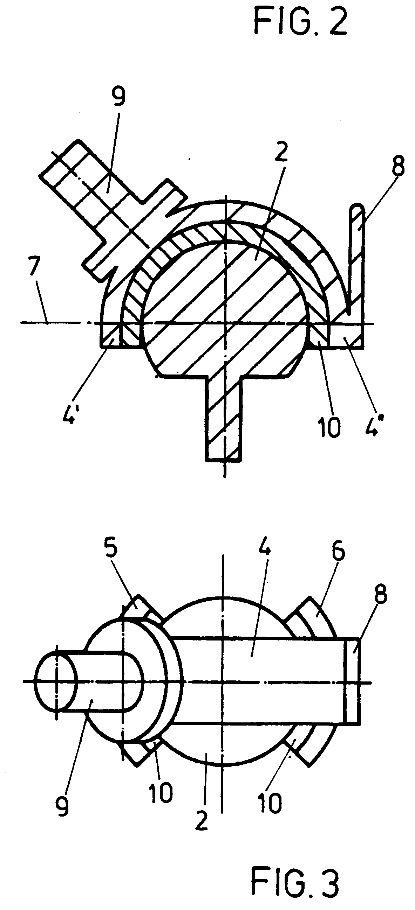

Auf seiten des Schulterblatts 21 ist in der angefrischten Cavitas glenoidalis 22 ein Lager 3 in Form eines Lagerkäfigs eingelassen. Der Lagerkäfig besteht aus einem ersten Ringsegment 4, dessen Krümmungsradius annäherungsweise derselbe ist wie jener des Kugelgelenkkopfes 2. Das erste Ringsegment weist eine Länge auf, die größer ist als der halbe Umfang des Kreisringes, dessen Teil es selbst ist. Dies bedeutet, daß die Enden 4' und 4'' des ersten Ringsegmentes (Fig. 2) den Kugelgelenkkopf 2 weiter als die Hälfte umfassen.

Zwei weitere Ringsegmente 5 und 6 sind an jeweils einem Ende 4' bzw. 4'' des ersten Ringsegmentes angeformt. Auch diese Ringsegmente 5 und 6 weisen einen Krümmungsradius auf, der annäherungsweise jenem des Kugelgelenkkopfes 2 entspricht. Dadurch, daß das erste Ringsegment 4 von der beschriebenen Länge ist, übergreifen die beiden anderen Ringsegmente 5 und 6 die Äquatorlinie 7 des im Lagerkäfig gehaltenen Kugelgelenkkopfes 2.

Im gezeigten Ausführungsbeispiel ist an dem ersten Ringsegment 4 außen im Bereich des Ringsegmentes 6 ein etwa tangential angesetzter Flansch 8 angeformt, der als Arretierungsmittel für den Lagerkäfig am Schulterblatt sorgt. Vorzugsweise geht man während der Operation so vor, daß der Lagerkäfig in die angefrischte Cavitas glenoidalis gesetzt wird und dreht den Lagerkäfig 3 dann über das Acromion 23, so daß ein regelrechter Klemmsitz des Lagerkäfigs erzielt wird. Im gezeigten Ausführungsbeispiel weist der Lagerkäfig 3 darüber hinaus einen radial nach außen weisenden Verankerungszapfen 9 auf, der in eine entsprechend präparierte Bohrung im Schulterblatt gesetzt ist. Dieser Zapfen 9 sorgt für eine zusätzliche Fixation des Lagerkäfigs 3.

Das Ausführungsbeispiel gemäß den Figuren 2 und 3 veranschaulicht, daß die dem Kugelgelenkkopf 2 zugewandten Flächen des Lagerkäfigs 3 jeweils mit einem Inlay 10 belegt sind. Dieses Inlay ist so elastisch, daß der Kugelgelenkkopf 2 unter elastischer Verformung der Inlays in den Kugelkäfig 3 eingedrückt werden kann.

Vorzugsweise besteht der Lagerkäfig 3 aus körperverträglichem Material, das im übrigen über eine ausreichende Festigkeit verfügen muß. Die Inlays 10 können beispielsweise aus hochverdichtetem Polyethylen bestehen.

Wesentlich ist in jedem Falle, daß der Kugelgelenkkopf 2 in den Lagerkäfig 3 unter elastischer Verformung desselben bzw. der Inlays gedrückt werden kann. Nach der entsprechenden Aufspreizung des Lagerkäfigs schnappen gewissermaßen die beiden anderen Ringsegmente 5 und 6 über den Kugelgelenkkopf 2 und sichern ihn gegen ein Luxieren.

## Patentansprüche

1. Schultergelenk-Endoprothese, bestehend aus einem im Humerus (20) zu verankernden Stielteil (1), einem mit dem proximalen Ende des Stielteils (1) verbindbaren Kugelgelenkkopf (2) und mit einem in der präparierten natürlichen Gelenkpfanne (22) am Schulterblatt (21) fixierbaren, als Lagerkäfig ausgebildeten Lager (3) für den Kugelgelenkkopf (2), dadurch gekennzeichnet, daß das Lager (3) aus im wesentlichen drei Ringsegmenten (4, 5, 6) besteht, von denen das erste Ringsegment (4) eine Bogenlänge aufweist, die größer ist als der halbe Umtang des Kreisringes, dessen Teil es selbst ist, und von denen die beiden anderen Ringsegmente (5, 6) an den Enden (4', 4'') des ersten Ringsegmentes (4) angebracht sind, derart, daß sie die Äquatorlinie (7) des im Kugelkäfig gehaltenen Kugelgelenkkopfes (2) zumindest teilweise übergreifen, wobei die Radien der Ringsegmente (4, 5, 6) und des Kugelgelenkkopfes (2) im wesentlichen gleich sind.

2. Schultergelenk-Endoprothese nach Anspruch 1, bei der die dem Kugelgelenkkopf (2) zugewandten Flächen des Lagerkäfigs (3) jeweils mit einem Inlay (10) aus reibungsarmem Material belegt sind, das so elastisch ist, daß der Kugelgelenkkopf (2) unter elastischer Verformung der Inlays in den Kugelkäfig (3) eingedrückt werden kann.

3. Schultergelenk-Endoprothese nach Anspruch 2, bei der die Inlays der beiden anderen Ringsegmente (5,6) auf ihrer Außenfläche eine Nut aufweisen, in die ein offener Federring eingelegt ist.

4. Schultergelenk-Endoprothese nach Anspruch 3, bei der die beiden anderen Ringsegmente (5, 6) auf ihrer Innenseite eine Nut aufweisen, in die der Federring zu liegen kommt.

5. Schultergelenk-Endoprothese nach einem der Ansprüche 1 bis 4, bei der am ersten Ringsegment (4) außen im Bereich des einen anderen Ringsegments (6) ein in etwa tangential angesetzter Flansch (8) als Arretierungsmittel des Lagerkäfigs (3) am Schulterblatt angeformt ist.

6. Schultergelenk-Endoprothese nach einem der Ansprüche 1 bis 5, bei der von dem ersten Ringsegment (4) radial nach außen weisend ein Verankerungszapfen (9) für die Fixierung des Lagerkäfigs (3) am Schulterblatt vorgesehen ist.

## Claims

1. Shoulder joint endoprosthesis consisting of a stem part (1) to be anchored in the humerus (20), a ball joint head (2) which can be connected to the proximal end of the stem part (1) and having a bearing (3) for the ball joint head (2) designed as a bearing cage and which can be fixed to the shoulder blade (21) in the prepared natural joint socket (22), characterised in that the bearing (3) consists of essentially three annular segments (4, 5, 6), of which the first annular segment (4) has an arc length which is greater than half the periphery of the circular ring, of which it is part, and of which the two other annular segments (5, 6) are attached to the ends (4', 4'') of the first annular segment (4) such that they at least partly engage over the equatorial line (7) of the ball joint head (2) retained in the ball cage, wherein the radii of the annular segments (4, 5, 6) and of the ball joint head (2) are essentially the same.

2. Shoulder joint endoprosthesis according to claim 1, in which the surfaces of the bearing cage (3) facing the ball joint head (2) are lined in each case with an inlay (10) of low-friction material which is so resilient that the ball joint head (2) may be pressed into the ball cage (3) with resilient deformation of the inlay.

3. Shoulder joint endoprosthesis according to claim 2, in which the inlays of the two other annular segments (5, 6) have on their outer surface a groove into which an open spring ring is inserted.

4. Shoulder joint endoprosthesis according to claim 3, in which the two other annular segments (5, 6) have on their inner side a groove into which the spring ring comes to rest.

5. Shoulder joint endoprosthesis according to one of claims 1 to 4, in which a flange (8) placed approximately tangentially on the first annular segment (4) is moulded-on externally in the region of the other annular segment (6) as a locking means for the bearing cage (3) on the shoulder blade.

6. Shoulder joint endoprosthesis according to one of claims 1 to 5, in which an anchoring pin (9) pointing radially outwards from the first annular segment (4) and for fixing the bearing cage (3) is provided on the shoulder blade.

## Revendications

1. Endoprothèse de l'articulation de l'épaule, constituée par une partie en forme de tige (1), qui doit être ancrée dans l'humérus (20), une tête d'articulation sphérique (2), qui peut être reliée à l'extrémité proximale de la partie en forme de tige (1), et un palier (3) pour la tête d'articulation sphérique (2), qui peut être fixé dans le coussinet d'articulation naturel préparé (22) présent sur l'omoplate (21) et est agencé sous la forme d'une cage de palier, caractérisée en ce que le palier (3) est constitué par essentiellement trois segments annulaires (4,5,6), parmi lesquels le premier segment annulaire (4) possède une longueur d'arc qui est supérieure à la moitié de la circonférence de l'anneau circulaire, dont la partie constitue ce segment lui-même, et parmi lesquels les deux autres segments annulaires (5,6) sont disposés sur les extrémités (4',4'') du premier segment annulaire (4) de telle sorte qu'il s'engage au moins partiellement au-delà de la ligne équatoriale (7) de la tête d'articulation sphérique (2) retenue dans la cage sphérique, les rayons des segments annulaires (4,5,6) et de la tête d'articulation sphérique (2) étant essentiellement égaux.

2. Endoprothèse de l'articulation de l'épaule selon la revendication 1, dans laquelle les surfaces, tournées vers la tête d'articulation sphérique (2), de la cage de palier (3) sont garnies respectivement par un insert (10) formé d'un matériau présentant un faible coefficient de frottement et qui est suffisamment élastique pour que la tête d'articulation sphérique (2) puisse être repoussée dans la cage sphérique (3) moyennant une déformation élastique de l'insert.

3. Endoprothèse de l'articulation de l'épaule selon la revendication 2, dans laquelle les inserts des deux autres segments annulaires (5,6) possèdent, sur leur surface extérieure, une rainure dans laquelle est insérée une bague élastique ouverte.

4. Endoprothèse de l'articulation de l'épaule selon la revendication 3, dans laquelle les deux autres segments annulaires (5,6) possèdent sur leur face intérieure une rainure, dans laquelle vient s'appliquer la bague élastique.

5. Endoprothèse de l'articulation de l'épaule selon l'une des revendications 1 à 4, dans laquelle une bride (8) montée approximativement tangentiellement est formée en tant que moyen d'arrêt de la cage de palier (3) sur l'omoplate, sur le premier segment annulaire (4) à l'extérieur dans la zone d'un autre segment annulaire (6).

6. Endoprothèse de l'articulation de l'épaule selon l'une des revendications 1 à 5, dans laquelle un téton d'ancrage (9) servant à fixer la cage de palier (3) sur l'omoplate est prévu en étant dirigé radialement vers l'extérieur, sur le premier segment annulaire (4).
